# EUROPEAN PATENT APPLICATION

(11) **EP 2 022 433 A1**
(43) Date of publication of application: **11.02.2009**
(21) Application number: 06756731.3
(22) Date of filing: 30.05.2006
(51) Int. Cl.: A61B 19/00, A61B 1/00

(54) **ENDOSCOPE TREATMENT SYSTEM**

(71) Applicant: Olympus Medical Systems Corp., Tokyo 151-0072 (JP)
(72) Inventor: HONDA, Kazuki, c/o Olympus Medical Systems Corp., Tokyo 151-0072 (JP); KURA, Yasuhito;c/o Olympus Medical Systems Corp., Tokyo 151-0072 (JP); NISHIIE, Takehiro, c/o Olympus Med. Systems Corp., Tokyo 151-0072 (JP); MURAKAMI, K., c/o Olympus Medical Systems Corp., Tokyo 151-0072 (JP); ONUKI, Yoshio, c/o Olympus Medical Systems Corp., Tokyo 151-0072 (JP); ICHIKAWA, Hiroaki, c/o Olympus Med. Systems Corp., Tokyo 151-0072 (JP); KOMIYA, Takaaki, c/o Olympus Med. Systems Corp., Tokyo 151-0072 (JP)
(74) Representative: von Hellfeld, Axel
(86) International application number: PCT/JP2006/310735
(87) International publication number: WO 2007/138674

(57) **Abstract**

An endoscope treatment system includes: an advancing and retracing device which electromotively advances and retracts a treatment instrument insertion portion of a treatment instrument to be introduced in a treatment instrument insertion channel through a treatment instrument opening opened at a mouth ring portion of an endoscope; and a connecting tube including a tube main frame to which the treatment instrument insertion portion is inserted and an attaching portion provided at one end portion of the tube main frame, while the attaching portion is arranged at the mouth ring portion or the treatment instrument insertion portion integrally.

## Description

### Technical Field

The present invention relates to an endoscope treatment system which is provided with an endoscope and an electromotive treatment instrument insertion portion advancing and retracting device that electromotively moves a treatment instrument insertion portion of a treatment instrument, which is to be used in combination with the endoscope, to let the treatment instrument insertion portion of the treatment instrument advance and retract.

### Background Art

In recent years, endoscopes have come to be used widely in the field of medicine. An endoscope is configured as including an elongated insertion portion and an operation portion which is provided at a proximal end of the insertion portion. Generally, a bendable bending portion is provided at a distal end side of the elongated insertion portion. The operation portion is provided with a knob for carrying out a bending operation of the bending portion, and various switches, etc. for conducting a variety of operations with respect to endoscope functions.

With respect to the endoscope used in the field of medicine, the insertion portion is to be inserted inside a body cavity of a test subject at the time when internal organs inside the body cavity are to be examined. Moreover, with respect to the endoscope, a variety of treatments can be performed by introducing the treatment instrument inside the body cavity through a treatment instrument channel provided in the insertion portion.

In a case of inserting the treatment instrument inside the treatment instrument channel of the endoscope, an operator holds a sheath (an insertion portion of a treatment instrument) and manually inserts the sheath into the treatment instrument channel. For the operator, the manual inserting operation of the sheath is a time-consuming burdensome operation. This is because a sheath portion that could measure up to 2 meters is difficult to be inserted without being buckled, and because attention has to be paid in order to prevent the sheath portion from touching unclean areas.

In order to resolve such problem, Japanese Patent Application Laid-Open Publication No. 2005-152502 discloses an endoscope treatment instrument inserting and withdrawing system (hereinafter to be referred to as the inserting and withdrawing system). The inserting and withdrawing system has a treatment instrument unit which is internally equipped with an insertion portion of a treatment instrument. The treatment instrument unit is to be arranged integrally with the endoscope while being in a sate of having a forceps opening connecting member of the treatment instrument unit connected to a forceps opening of the endoscope and a motor connecting portion of the treatment instrument unit connected to a connecting portion of the endoscope.

In the inserting and withdrawing system, a motor is driven at the time of inserting and withdrawing the treatment instrument. At this time, the operator, etc. does not have to support the treatment instrument and can easily do the insertion and withdrawal of the treatment instrument without giving too much burden on the treatment instrument. In the treatment instrument unit, a manual operation portion is provided. At the time of performing manual operation, the operator is to grasp and squish a knob of the manual operation portion. Thereby, a tubular portion of the knob will shrink to hold the insertion portion, thus enabling an advancing and retracting operation of the insertion portion by manual operation.

Therefore, by using the manual operation portion, the operator can perform insertion and withdrawal of the insertion portion by manual operation where necessary. However, some operators would like to grasp the insertion portion of the treatment instrument by their own fingers in performing the inserting operation or the withdrawing operation, etc. where necessary.

The present invention is brought about in view of the circumstances as described above, and the object of the present invention is to provide an endoscope treatment system which is capable of performing an operation for inserting and withdrawing a treatment instrument insertion portion of a treatment instrument with respect to a treatment instrument channel of an endoscope using an electromotive treatment instrument insertion portion advancing and retracting device, and which is capable of letting the electromotive treatment instrument insertion portion advancing and retracting device stop where necessary to let an operator, etc. perform insertion and withdrawal of the insertion portion with the fingers.

### Disclosure of Invention

### Means for Solving the Problem

An endoscope treatment system according to the present invention includes: an advancing and retracing device which electromotively advances and retracts a treatment instrument insertion portion of a treatment instrument to be introduced in a treatment instrument insertion channel through a treatment instrument opening opened at a mouth ring portion of an endoscope; and a connecting tube including a tube main frame to which the treatment instrument insertion portion is inserted and an attaching portion provided at one end portion of the tube main frame, while the attaching portion is arranged at the mouth ring portion or the treatment instrument insertion portion integrally.

### Brief Description of the Drawings

Fig. 1 is diagram illustrating an endoscope treatment system;
Fig. 2 is a diagram illustrating a configuration inside an electromotive advancing and retracting device;
Fig. 3 is a cross-section diagram of Fig. 2 take at line III-III;
Fig. 4 is a diagram illustrating a connecting tube in an elongated state as being connected to a mouth ring portion of a first attaching portion;
Fig. 5 is a diagram illustrating the connecting tube in a contracted state where the first attaching portion after being removed from the mouth ring portion is attached to a sheath in a way such that the sheath will be exposed;
Fig. 6 is a diagram illustrating operation of the connecting tube;
Fig. 7 is a diagram illustrating another configuration example of the first attaching portion;
Fig. 8 is a diagram illustrating a state in which the first attaching portion is fixed to the sheath integrally;
Fig. 9 is a diagram showing a state in which the first attaching portion is attached and detached to a connecting member of the mouth ring portion while the first attaching portion is in the sate of being fixed to the sheath integrally;
Fig. 10 is a diagram illustrating still another configuration example of the first attaching portion;
Fig. 11 is a diagram illustrating a rigid connecting tube in which a forceps plug is arranged at a mouth ring configuration member provided in a rigid tube main frame;
Fig. 12 is a diagram illustrating a rigid connecting tube having a connecting portion for connecting a plurality of rigid tubes; and
Fig. 13 is a diagram illustrating an advancing and retracting device having a space portion where a connecting portion is arranged in a slidable manner, and operation of the space portion.

### Best Mode for Carrying Out the Invention

In the following, an embodiment of the present invention will be described with reference to the drawings.

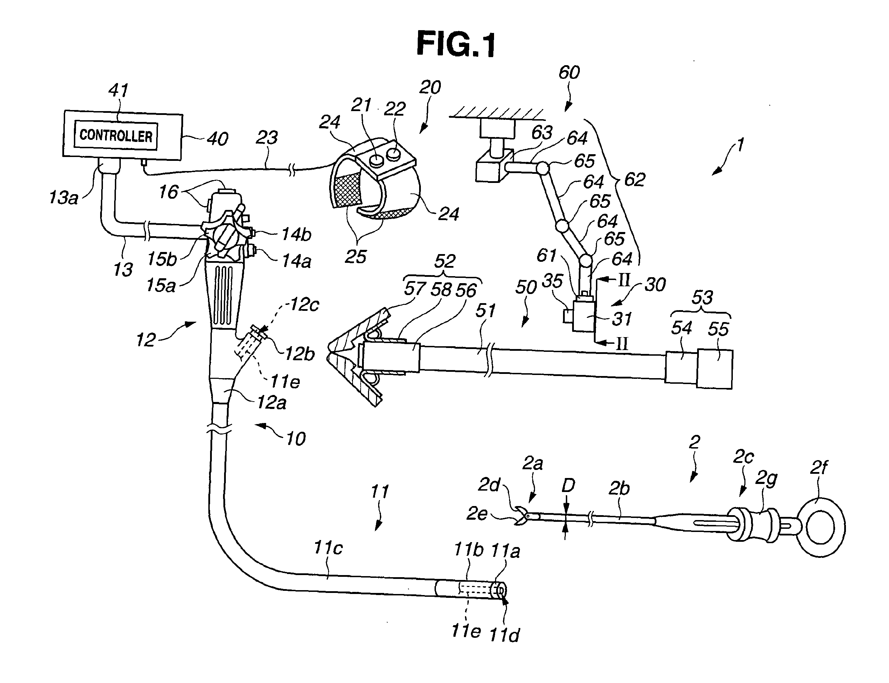

One embodiment of an endoscope treatment system according to the present invention will be described with reference to Figs. 1 to 6.

As shown in Fig. 1, an endoscope treatment system 1 is configured as mainly including a treatment instrument 2, an endoscope 10, an operation instruction switch 20, an electromotive treatment instrument insertion portion advancing and retracting device (hereinafter to be referred to as advancing and retracting device) 30 which is an advancing and retracting device to be operated electromotively, a controlling device 40 provided with a controller 41, and a connecting tube 50.

In the present embodiment, a box chassis 31 of the advancing and retracting device 30 is capable of being attached to an attaching portion 61 of an arm device 60 integrally through screw connection, etc.

The arm device 60 is provided with an arm portion 62. One end of the arm portion 62 is attached to a rotation holding portion 63 in a turnable way, the rotation holding portion 63 being provided on a ceiling, etc. of an operating room, etc. The arm portion 62 is configured as including, for instance, a plurality of arm members 64 with different lengths and joint portions 65 each of which connects adjacent arm members 64 in a turnable way. The arm portion 62 has a configuration which is capable of holding a placement position of the advancing and retracting device 30, and which is capable of freely moving the placement position of the advancing and retracting device 30 with a small amount of power.

The controlling device 40 is provided with a light source device and a video processor in the inside in addition to the controller 41. The controlling device 40 can be connected by a liquid crystal displaying device (not shown), etc. for displaying an endoscope image.

The operation instruction switch 20 is provided with operation buttons 21 and 22. An electric cable 23 is extending outward from the operation instruction switch 20. An end portion of the electric cable 23 has a configuration that is detachable to the controlling device 40. That is, in the present embodiment, the operation instruction switch 20 is of a wired system.

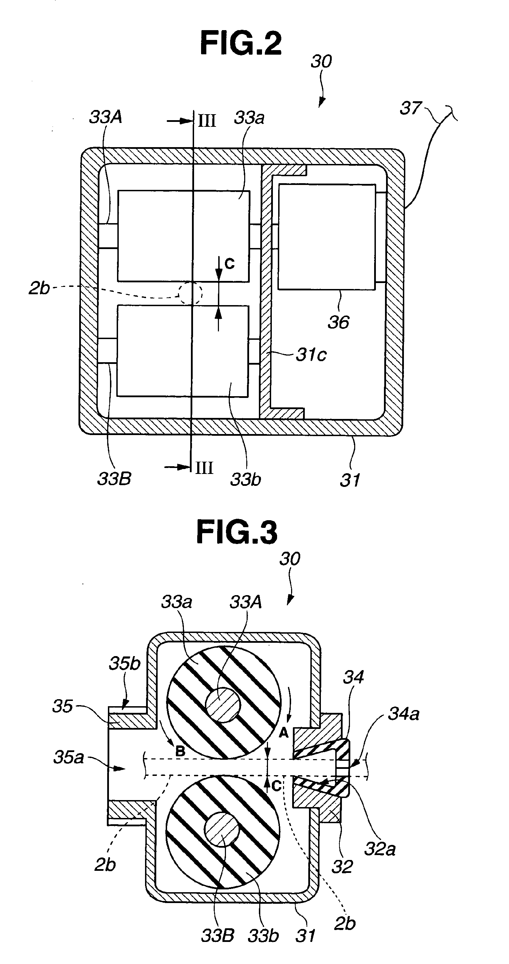

While an operator is pressing the operation button 21, for instance, the operation instruction switch 20 outputs a sheath advancing movement instruction signal (hereinafter to be abbreviated to advance signal) to the controlling device 40 via the electric cable 23. When the controlling device 40 receives the advance signal, the controlling device 40 outputs a control signal from the controller 41 to the advancing and retracting device 30, the control signal bringing a roller 33a to rotate in a direction of arrow A as shown in Fig. 3.

On the other hand, while the operator is pressing the operation button 22, for instance, the operation instruction switch 20 outputs a sheath retracting movement instruction signal (hereinafter to be abbreviated to retract signal) to the controlling device 40 via the electric cable 23. When the controlling device 40 receives the retract signal, the controlling device 40 outputs a control signal from the controller 41 to the advancing and retracting device 30, the control signal bringing the roller 33a to rotate in a direction of arrow B as shown in Fig. 3.

Reference numeral 24 denotes a detachable belt which is provided integrally with the operation instruction switch 20. A Velcro tape 25 is arranged at the detachable belt 24. Therefore, the operation instruction switch 20 can be attached detachably to an arbitrary place of an operation portion 12, for instance. The operation instruction switch 20 is not limited to being of the wired system but could be of a wireless system.

In the present embodiment, the treatment instrument 2 is biopsy forceps, for instance. The biopsy forceps 2 are configured as including, in the order from a side of a distal end, a tissue collecting portion 2a which is a functional portion, an elongated sheath 2b which is a treatment instrument insertion portion, and a handle portion 2c which is an operation portion. The tissue collecting portion 2a is arranged at the distal end of the sheath 2b. The tissue collecting portion 2a is provided with a pair of biopsy cups 2d and 2e, where the biopsy cups 2d and 2e are configured to open/close freely with respect to each other. An operation wire (not shown) is being inserted in the sheath 2b of the biopsy forceps 2.

The handle portion 2c is arranged at a proximal end of the sheath 2b. The handle portion 2c is configured as including a finger hook ring 2f and a slider 2g. The finger hook ring 2f has a hole portion where a user can place the user's thumb, for instance. The slider 2g is provided with a pair of flange portions at a midway portion thereof where the user can place the user's middle finger and ring finger. The operation wire moves forward and backward with an operation at the handle portion 2c.

Specifically, the operation wire moves as the operator moves the slider 2g forward and backward along an axis of the handle portion 2c. Then the cups 2d and 2e which configure the tissue collecting portion 2a will open and close according to the forward and backward movement of the slider 2g.

Meanwhile, the treatment instrument is not limited to the biopsy forceps 2 but could be a high-frequency snare, basket forceps, etc.

The endoscope 10 is configured as including an insertion portion 11, an operation portion 12 and a universal code 13. The operation portion 12 also serves as a grasping portion and is arranged at a proximal end side of the insertion portion 11. The universal code 13 is extending from a side of the operation portion 12, and a connector 13a provided at a proximal end of the universal code 13 is connected to the controlling device 40 detachably.

The insertion portion 11 is configured as including, in the order from a side of a distal end, a rigid distal end portion 11 a, a bendable bending portion 11b, and a flexible tube portion 11c having flexibility, which are being juncturally connected. The operation portion 12 has a bend preventing portion 12a arranged being connected with a proximal end of the flexible tube portion 11c. The operation portion 12 is provided with an air and water supply button 14a for supplying air and water, a suction button 14b for conducting suctioning, bending knobs 15a and 15b for conducting a bending operation on the bending portion 11b, and various switches 16 for controlling an endoscope image displayed on a screen of a displaying device, the endoscope image being picked up by image pickup means such as CCD, etc. arranged at the distal end portion 11a.

Moreover, the operation portion 12 has a mouth ring portion 12b to which the connecting tube 50, which will be described later on, can be attached detachably. The endoscope 10 has a treatment instrument channel 11e which communicates between a treatment instrument opening 12c arranged at the mouth ring portion 12b and a distal end opening 11d of the distal end portion 11a. Into the treatment instrument channel 11e are inserted a functional portion and a treatment instrument insertion portion of a treatment instrument, such as the biopsy forceps 2 having the tissue collecting portion 2a and the sheath 2b, a high-frequency snare, and basket forceps.

The advancing and retracting device 30 will now be described with reference to Fig. 1, Fig. 2 and Fig. 3.

The advancing and retracting device 30 is provided with two turnable rollers 33a and 33b inside the box chassis 31. The box chassis 31 is configured as having a sheath insertion portion 32 being arranged on a side of one of the facing surfaces, and into the sheath insertion portion 32 the sheath 2b of the biopsy forceps 2 is inserted. The sheath insertion portion 32 has a communicating hole 32a. The communicating hole 32a provides a forceps plug 34 formed of an elastic member. A slit 34a is being formed in the forceps plug 34. The sheath 2b of the biopsy forceps 2 is inserted into the slit 34a. The slit 34a is in a substantially tightly sealed state before the sheath 2b is inserted thereto.

A connecting portion 35 which is a convex portion is arranged on a side of the other surface of the box chassis 31. The connection portion 35 arranges an insertion portion inserting hole 35a through which the tissue collecting portion 2a and the sheath 2b, which have been inserted through the slit 34a, are passed. Male screws 35b are formed on an outer periphery of the connecting portion 35. An attaching portion 53 of the connecting tube 50, which will be described later on, is arranged on the male screws 35b through screw connection.

The respective rollers 33a and 33b are configured with resin members or rubber members. The rollers 33a and 33b are fixed to turning axes 33A and 33B, respectively, integrally. The rotating axis 33A is a drive axis which is turned by a motor 36 arranged inside the box chassis 31. On the other hand, the rotating axis 33B is a driven axis which is arranged inside the box chassis 31 in a turnable manner. An electric cable 37 extending from the advancing and retracting device 30 is connected to a connector, which is not shown, of the attaching portion 61 that configures the arm device 60. Thereby, the advancing and retracting device 30 is electrically connected with the controlling device 40 through a distribution cable (not shown) arranged inside the arm device 60, a distribution cable (not shown) arranged in the operating room, etc.

The rotating axis 33A and the rotating axis 33B are supported by side walls and a supporting plate 31 c of the box chassis 31 in a way such that the turning axes 33A and 33B become parallel with each other and such that the turning axes 33A and 33B are rendered turnable. An interval between roller surfaces of the rollers 33a and 33b fixed to the turning axes 33A and 33B, respectively, is a distance "C". The interval "C" is set to a value that is smaller than an outside diameter "D" of the sheath 2b. Thereby, an outer surface of the sheath 2b inserted through the slit 34a is held and wedged between the roller surfaces of the two rollers 33a and 33b with a predetermined pressing force.

The motor 36 is controlled of its drive by the advance signal and the retract signal outputted from the operation buttons 21 and 22 provided at an operation instruction switch 20, respectively. Specifically, the operator presses the operation button 21, for example, under the condition that the sheath 2b is held and wedged between the roller surfaces of the rollers 33a and 33b in a way shown by dashed lines, for instance. Then the rotating axis 33A will rotate in the direction of arrow A by the motor 36, and the roller 33a will be rotated in accordance with the rotation of the rotating axis 33A. Thereby, the sheath 2b being held and wedged between the rollers 33a and 33b will advance forward.

The connecting tube 50 will now be described with reference to Fig. 1, Fig. 4 and Fig. 5.

As shown in the diagrams, the connecting tube 50 is configured as including a tube main frame 51, an endoscope side attaching portion (hereinafter to be referred to as the first attaching portion) 52 which is configured with a rigid member such as a metal member, for instance, and an advancing and retracting device side attaching portion (hereinafter to be referred to as the second attaching portion) 53 which is configured with a metal member. The attaching portions 52 and 53 can also be configured with rigid resin members instead of the metal members.

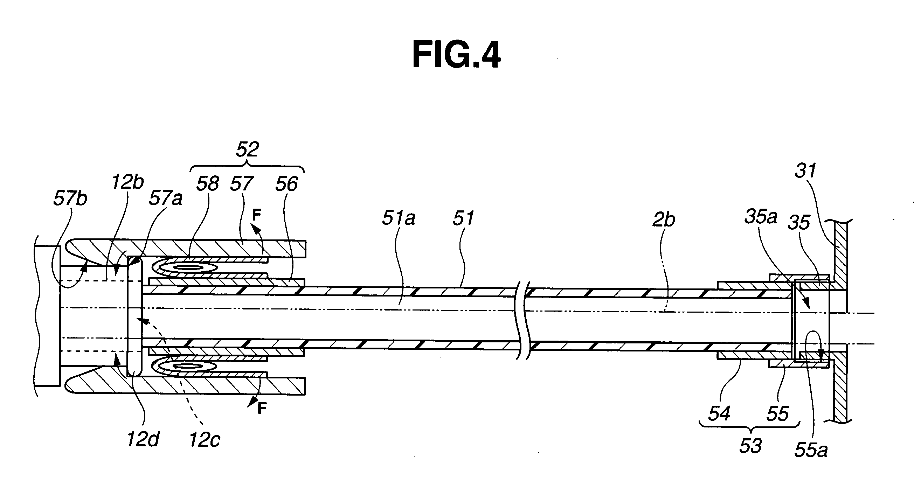

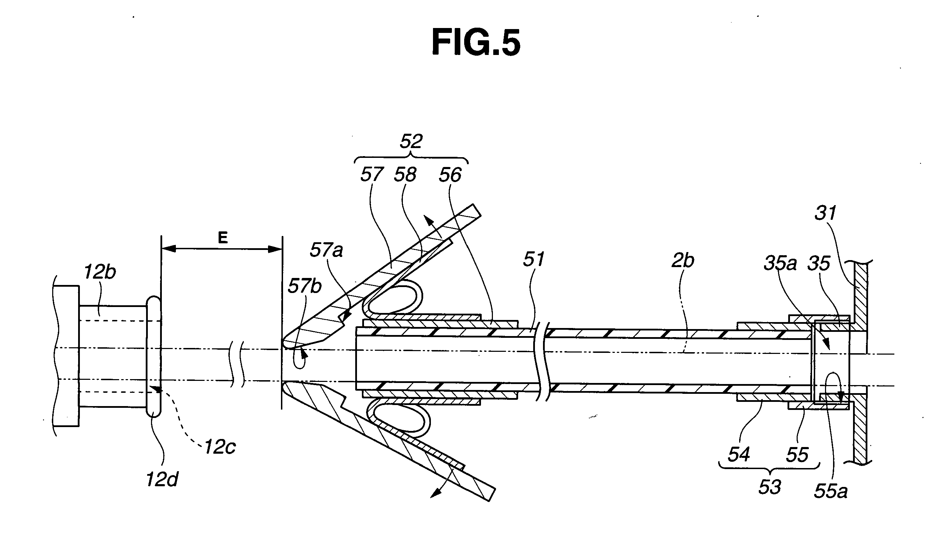

The tube main frame 51 prevents the sheath 2b from contacting unclean areas. That is, the tissue collecting portion 2a and the sheath 2b are inserted to an inner hole 51 a of the tube main frame 51. The tube main frame 51, for instance, is a stretchable elastic tube which is configured as having a predetermined length. Specifically, the length of the tube main frame 51 changes by as much as a predetermined length "E" between an elongated state as shown in Fig. 4 and a contracted state as shown in Fig. 5. In other words, as shown by chain double dashed lines, the sheath 2b is to be exposed by as much as the length "E" as the tube main frame 51 is transformed from the elongated state to the contracted state. Therefore, the operator, etc. is capable of grasping the sheath 2b where it is exposed by as much as the length "E" with the fingers and move the sheath 2b to let the sheath 2b be advanced and retracted by manual operation.

The first attaching portion 52 is arranged at one end portion of the tube main frame 51, and the second attaching portion 53 is arranged at the other end portion of the tube main frame 51. The second attaching portion 53 is configured with an attaching portion main frame 54 and a rotating ring 55. The attaching portion main frame 54 is a tubular member fixed to the other end portion of the tube main frame 51 integrally by adhesion, for example. The rotating ring 55 is arranged on an outer periphery of attaching portion main frame 54 in a turnable manner. The rotating ring 55 is provided with female screws 55a, which is screw-connected with the male screws 35b, within an inner periphery thereof. Accordingly, the second attaching portion 53 can be attached to the advancing and retracting device 30 integrally by having the female screws 55a of the rotating ring 55 screw-connected with the male screws 35b provided in the connecting portion 35 of the advancing and retracting device 30.

The first attaching portion 52 is configured as including an attaching portion main frame 56, a pair of holding members 57 and an urging member 58. The attaching portion main frame 56 is a tubular member fixed to one end portion of the tube main frame 51 integrally by adhesion, for example. In this fixed state, an end face of the tube main frame 51 sticks out from an end face of the attaching portion main frame 56 by a predetermined amount.

The holding members 57 have a half-pipe shape, and each includes a first holding portion 57a and a second holding portion 57b. The first holding portion 57a is a nail-shaped portion which is arranged locked such that a convex portion 12d of the mouth ring portion 12b can hook into the first holding portion 57a. On the other hand, the second holding portion 57b is a peripheral surface portion which is arranged coherent to an outer surface of the sheath 2b of the biopsy forceps 2 due to a pressing force of the urging member 58. The urging member 58 urges each of the holding members 57 in a direction of arrow F which is a direction in which a proximal end side portion of the holding member 57 is opened. The urging member 58 is a coil spring, a plate spring or the like, which is arranged on the attaching portion main frame 56 and the urging member 58 integrally through welding, for instance.

As shown in Fig. 4, one end face of the tube main frame 51 is arranged coherent to an end face of the mouth ring portion 12b, while the first holding portions 57a of the holding members 57 are in a state of being locked at the mouth ring portion 12b.

Moreover, as shown in the same diagram, in a case of removing the holding members 57 from the mouth ring portion 12b under the circumstance where the first holding portions 57a of the respective holding members 57 are locked at the mouth ring portion 12b, the operator, etc. pushes and moves the proximal end side portions of the respective holding members 57 toward the attaching portion main frame 56 against an urging force of the urging member 58. Then the first holding portions 57a will make a diameter wider than an external diameter of the convex portion 12d. Thereby, the holding members 57 can be removed from the mouth ring portion 12b by one-touch operation.

Furthermore, in the above description, the operator, etc. is supposed to be able to grasp with the fingers the sheath 2b where it is exposed by as much as the length "E", as shown by the chain double dashed lines, and move the sheath 2b to let the sheath 2b be advanced and retracted by manual operation. However, the distance that the first attaching portion 52 can be moved is not limited to the length "E". In other words, the second holding portions 57b can also be arranged to be coherent to the outer surface of the sheath 2b right after the holding members 57 are removed from the mouth ring portion 12b, without having to expose the sheath 2b by as much as the length "E". Thereby, the operator, etc. can grasp the first attaching portion 52 and move the sheath 2b along with the first attaching portion 52 to let the sheath 2b be advanced and retracted by manual operation.

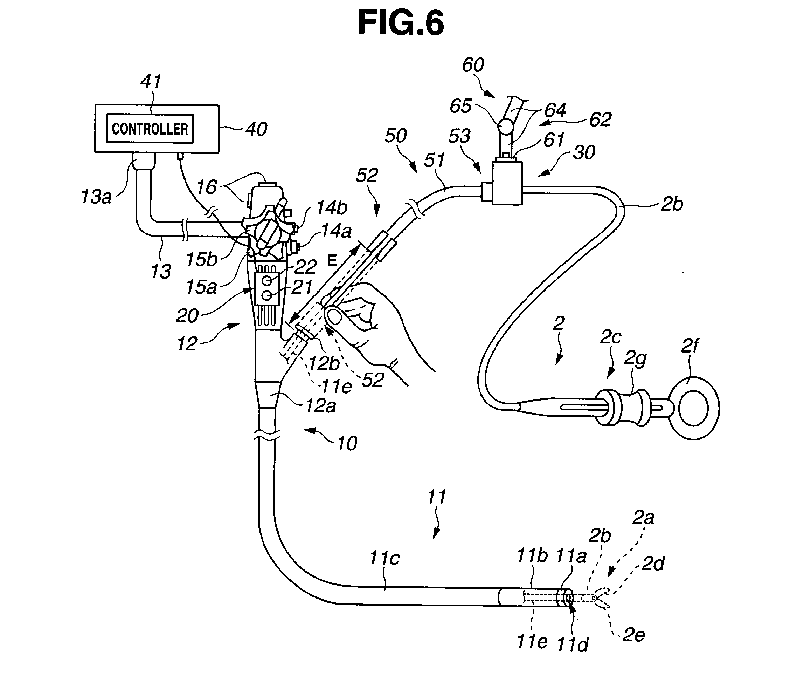

Operation of the endoscope treatment system 1 as configured in the above-described manner will be described with reference to Fig. 6.

In using the endoscope treatment system 1 in an operation, first of all, a staff prepares the biopsy forceps 2, for instance, which is the treatment instrument to be used in the operation, and the connecting tube 50 which has a length that corresponds with the treatment. Then the staff attaches the advancing and retracting device 30 to the attaching portion 61 of the arm device 60.

After that, the first attaching portion 52 of the connecting tube 50 is attached to the mouth ring portion 12b as shown by dashed lines, and the second attaching portion 53 of the connecting tube 50 is fixed to the connecting portion 35 of the advancing and retracting device 30 through screw-connection. At this point, a position of the advancing and retracting device 30 is adjusted by reshaping the arm portion 62 in order to let the tube main frame 51 be in the elongated state as shown in Fig. 4.

Moreover, the staff attaches the operation instruction switch 20 to the operation portion 12, for instance, and connects the electric cable 23 to the controlling device 40. After that, the staff inserts the tissue collecting portion 2a and the sheath 2b through the slit 34a of the forceps plug 34 provided in the advancing and retracting device 30.

Thereby, the sheath 2b will be held and wedged between the rollers 33a and 33b. At this point, the staff presses the operation button 21 of the operation instruction switch 20 in order to arrange the tissue collecting portion 2a in the vicinity of the distal end portion 11a.

Then the advance signal will be outputted from the operation instruction switch 20 to the controlling device 40, and the motor 36 inside the advancing and retracting device 30 will rotate on the basis of a control signal outputted from the controlling device 40, whereby the sheath 2b will start advancing forward. Then the sheath 2b is inserted inside the treatment instrument channel 11e through the inner hole 51a in the tube main frame 51 of the connecting tube 50 and the treatment instrument opening 12c of the mouth ring portion 12b. After that, the staff properly operates the operation buttons 21 and 22 in order to arrange the tissue collecting portion 2a at a predetermined position in the vicinity of the distal end portion 11a.

In conducting tissue collection, the operator inserts the insertion portion 11 of the endoscope 10 toward a target site inside a body cavity of a test subject while monitoring an endoscope image. At this point, the operator conducts hand-side operations such as an inserting operation and a bending operation for bending the bending portion 11b, etc. while confirming the endoscope image displayed on a screen of the displaying device.

While the operator is conducting the hand-side operation, the operator will also operate the operation buttons 21 and 22 for the tissue collecting portion 2a to face off against the target site, so that the operator can smoothly conduct the tissue collection with the biopsy forceps 2. After that, the operator properly operates the handle portion 2c to have a tissue collected in the tissue collecting portion 2a. After the operator finishes the tissue collection, the operator will press the operation button 22 in order to set the tissue collecting portion 2a having collected the tissue apart from the target site by a predetermined distance, and then withdraws the sheath 2b.

At this time, the operator, etc. determines as to whether the tissue collected by the tissue collecting portion 2a should be retrieved by advancing and retracting the sheath 2b by an operation with the operation instruction switch 20 or the tissue collected by the tissue collecting portion 2a should be retrieved by advancing and retracting the sheath 2b by manual operation.

In the case when the operator, etc. determines that the tissue collected by the tissue collecting portion 2a should be retrieved by retracting the sheath 2b by the operation with the operation instruction switch 20, the operator, etc. will carry out a push operation on the operation button 22 while keeping a state in which the position of the insertion portion 11 is maintained.

Then the sheath 2b will start retracting, whereby the tissue collecting portion 2a is inserted to the treatment instrument channel 11e through the distal end opening 11d, after which the tissue collecting portion 2a is led to the outside passing through the treatment instrument opening 12c of the mouth ring portion 12b and via the inner hole 51a in the tube main frame 51 of the connecting tube 50, the insertion portion inserting hole 35a provided in the box chassis 31, in between the rollers 33a and 33b, and the slit 34a at the forceps plug 34 fixed to the box chassis 31.

After that, the operator or the staff retrieves the tissue collected by the tissue collecting portion 2a. Then, in a case of conducting additional collection of tissue, the tissue collecting portion 2a and the sheath 2b are introduced into the body cavity through the slit 34a of the forceps plug 34 once again by the same procedure as described above. By the time when the reinsertion is completed, since the position of the advancing and retracting device 30 and the position of the insertion portion 11 are still maintained at the same positions without being changed, the tissue collecting portion 2a will be arranged at a position apart from the tissue by the predetermined distance which is a position approximately the same as the position where the withdrawal started.

On the other hand, in the case when the operator, etc. determines that the tissue collected by the tissue collecting portion 2a should be retrieved by retracting the sheath 2b by manual operation, the operator, etc. will grasp the proximal end side portions of the holding members 57 in the first attaching portion 52 that configures the connecting tube 50 while keeping a state in which the position of the insertion portion 11 is maintained. Then the operator, etc. pushes the holding members 57 toward the attaching portion main frame 56 against the urging force of the urging member 58. Thereby, the first holding portions 57a will make a diameter wider than that of the convex portion 12d.

At this point, the operator, etc. removes the holding members 57 from the mouth ring portion 12b. Then, the tube main frame 51 of a stretchable state starts contracting in a direction of contraction. At this time, the operator, etc. retracts the first attaching portion 52 by as much as a distance "E" as shown by a solid line, for instance, and let the second holding portions 57b of the holding members 57 be arranged coherent to the outer surface of the sheath 2b by the urging force of the urging member 58.

Thereby, a part of the sheath 2b being inserted in the treatment instrument channel 11e comes to a state of being exposed from the mouth ring portion 12b. At this point, the operator, etc. directly grasps the sheath 2b or grasps the first attaching portion 52, as shown in the diagram. Then the operator, etc. withdraws the sheath 2b inside the treatment instrument channel 11e by manual operation. Thus, the tissue collecting portion 2a will be led to the outside from the treatment instrument opening 12c of the mouth ring portion 12b.

After that, the operator or the staff retrieves the tissue collected by the tissue collecting portion 2a. Then, in a case of conducting additional collection of tissue, the operator, etc. reinserts the tissue collecting portion 2a and the sheath 2b, which have been withdrawn, into the treatment instrument channel 11e via the treatment instrument opening 12c of the mouth ring portion 12b by manual operation so as to be able to collect tissues at multiple sites. At this time, the first attaching portion 52 is attached to the mouth ring portion 12b. By the time when the reinsertion is completed, since the position of the advancing and retracting device 30 and the position of the insertion portion 11 are still maintained at the same positions without being changed, the tissue collecting portion 2a will be arranged at a position which is a position approximately the same as the position where the withdrawal started.

In this way, the connecting tube is configured with the stretchable tube main frame, the first attaching portion which is arranged at one end portion of the tube main frame and which is attached to the mouth ring portion of the endoscope in a way detachable by one-touch operation, and the second attaching portion which is arranged at the other end portion of the tube main frame and which is attached to the advancing and retracting device integrally. The sheath being the treatment instrument whose advancing and retracting is operated by the advancing and retracting device is introduced into the treatment instrument channel of the endoscope via the connecting tube, under the condition that the first attaching portion is being attached to the mouth ring portion. Thereby, it is possible to unfailingly prevent the sheath, which is advanced and retracted by the advancing and retracting device, from contacting unclean areas in between the advancing and retracting device and the mouth ring portion.

Moreover, the mouth ring portion and the advancing and retracting device are connected by the connecting tube while having the stretchable tube main frame in the elongated state. Under this condition, the operator or the staff removes the first attaching portion from the mouth ring portion where necessary. Then the tube main frame in the elongated state will be deformed into a contracted state whereby a part of the sheath comes to a state of being exposed. That is, the sheath having been covered by the tube main frame can be brought to an exposed state while at the same time the operator or the staff is working on removing the first attaching portion from the mouth ring portion. Thereby, the operator or the staff will be able to grasp the exposed sheath and conduct advancement and retraction of the sheath by manual operation. Therefore, tissue collection at multiple sites can be done in a short period of time since the tissue collecting portion can be led outside through the treatment instrument opening of the mouth ring portion.

Furthermore, the holding members that configure the first attaching portion are provided with the first holding portions for attaching the first attaching portion to the mouth ring portion, and the second holding portions for letting the first attaching portion be arranged coherent with the sheath integrally. The respective holding portions are configured in a way such that the holding portions can be attached to the mouth ring portion and the outer periphery of the sheath, respectively, by the urging force of the urging member which urges the holding members. Therefore, swift switching between the holding state due to the first holding portions and the holding state due to the second holding portions can be made possible by moving the holding members against the urging force by the urging member. In addition, the first attaching portion being removed from the mouth ring portion is fixed to the sheath integrally. Thereby, at the time when the first attaching portion is removed from the mouth ring portion and the sheath is operated to advance and retract by manual operation, the manual operation will not be hindered by the first attaching portion.

Meanwhile, in the present embodiment, the tube main frame 51 is supposed to be configured with an elastic tube which is stretchable. However, the tube main frame 51 is not limited to being a stretchable elastic tube, but could be a kind of a bellows member which is rendered stretchable by an external force, or a kind of a bellows member which is contracted by an external force and is restored to an elongated state by its own elastic force.

Moreover, in the present embodiment, the connecting tube 50 is configured as including the first attaching portion 52 and the second attaching portion 53 in the tube main frame 51. However, the configuration of the connecting tube 50 is not limited to such configuration, but could be a configuration in which a lure mouth ring and the first attaching portion are arranged in the tube main frame 51. In this case, the lure mouth ring should be able to be attached to the mouth ring portion detachably, and the first attaching portion should be attached to a connecting portion, which is not shown, of the box chassis of the advancing and retracting device 30 in a way detachable by one-touch operation. Thereby, the operator, etc. can remove the first attaching portion on the side of the advancing and retracting device 30, where necessary, and conduct advancement and retraction of the sheath by manual operation.

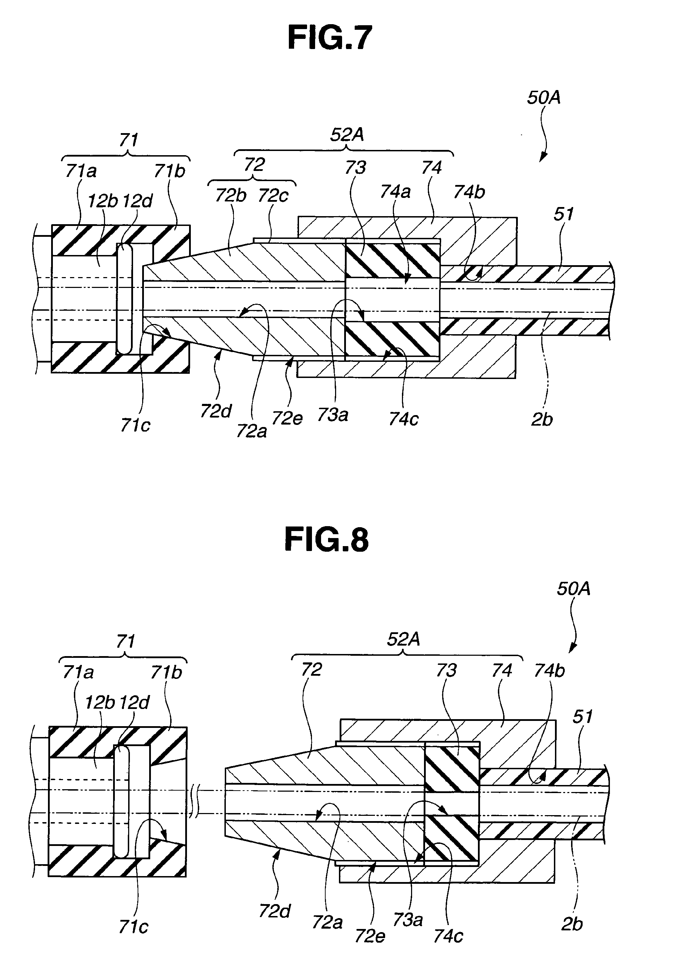

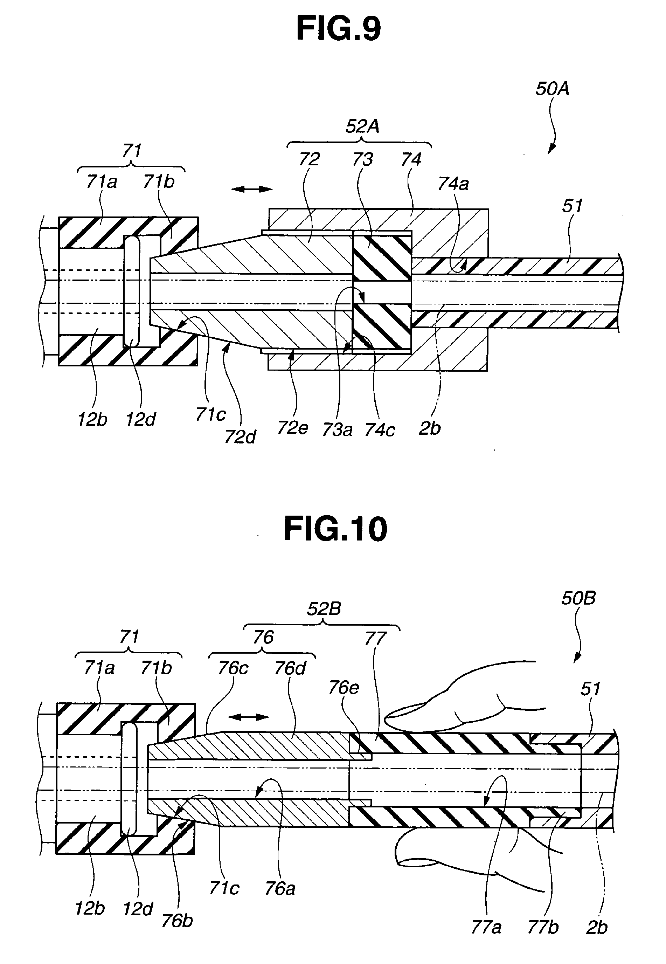

In addition, the configuration of the first attaching portion 52 which is detachable with respect to the mouth ring portion 12b is not limited to the above-described configuration that includes the attaching portion main frame 56, the holding members 57 and the urging member 58. That is, the first attaching portion 52A can be configured as a first attaching portion 52A as shown in Fig. 7, Fig. 8 and Fig. 9, or as a first attaching portion 52B as shown in Fig. 10, in whose configuration the side of the first attaching portion of a connecting tube 50A is connected to the mouth ring portion by press fitting. Specific configurations of the first attaching portions will be described in the following.

Another configuration example of the first attaching portion will now be described with reference to Fig. 7 and Fig. 8.

As shown in Fig. 7, the connecting tube 50A has the first attaching portion 52A at one end portion thereof. The first attaching portion 52A is configured as being detachable with respect to a connecting member 71 which is provided integrally with the mouth ring portion 12b. The first attaching portion 52A is configured as including a connecting portion main frame 72, a deforming member 73 and a fixing portion main frame 74.

The fixing portion main frame 74 is a tubular rigid member including a concave portion 74a and a communicating hole 74b. The communicating hole 74b is communicated with the concave portion 74a, and the communicating hole 74b is fixed to one end portion of the tube main frame 51 integrally by adhesion, for example. Female screws 74c are arranged on an inner periphery of the concave portion 74a.

The deforming member 73 is an elastic member configured as including a through hole 73a. A diameter of the through hole 73a is being previously set to be larger than a diameter of the sheath 2b considering the amount of expansion.

The connecting portion main frame 72 is provided with a through hole 72a whose diameter is being previously set to be larger than the diameter of the sheath 2b. The connecting portion main frame 72 is a tubular rigid member including a press fitting portion 72b having a taper face 72d and a pressing portion 72c of an approximately columnar shape. Male screws 72e which are screw-connected with the female screws 74c are arranged on an outer periphery of the pressing portion 72c.

On the other hand, the connecting member 71 is an elastic member configured in a cylindrical shape. The connecting member 71 is provided with a press fixing portion 71a and a press fit fixing portion 71b. The press fixing portion 71a has an elastic force that can bring the connecting member 71 to be arranged integrally with the mouth ring portion 12b. The connecting member 71 has the mouth ring portion 12b configured as having the press fixing portion 71a arranged fixed to the mouth ring portion 12b by the elastic force of the press fixing portion 71a. The press fit fixing portion 71b is provided with a taper hole 71c. The taper face 72d of the press fitting portion 72b is pressed into the taper hole 71c. Thereby, the first attaching portion 52A is arranged fixed to the mouth ring portion 12b integrally.

According to the configuration of the first attaching portion 52A, at the time of attaching the first attaching portion 52A to the mouth ring portion 12b, the operator grasps the fixing portion main frame 74 and presses the taper face 72d of the connecting portion main frame 72 into the taper hole 71c of the connecting member 71 which is being arranged fixed to the mouth ring portion 12b. Thereby, attachment of the first attaching portion 52A arranged at the distal end portion of the tube main frame 51 to the mouth ring portion 12b will be completed.

At the time of removing the first attaching portion 52A from the mouth ring portion 12b, the operator grasps the fixing portion main frame 74 and removes the taper face 72d of the connecting portion main frame 72 from the taper hole 71 c against the elastic force of the connecting member 71.

In this way, with the first attaching portion 52A, attachment or removal of the first attaching portion 52A, which is provided at one end portion side of the tube main frame 51, with respect to the mouth ring portion 12b can be done easily in one sequence of actions. Then a part of the sheath 2b can be brought to an exposed state by removing the first attaching portion 52A from the mouth ring portion 12b.

In the present embodiment, the first attaching portion 52A in a sate of being removed from the mouth ring portion 12b can be fixed to the sheath 2b integrally by letting the connecting portion main frame 72 gradually become constricted.

Specifically, the connecting portion main frame 72 is gradually constricted at the time of fixing the first attaching portion 52A having been removed from the mouth ring portion 12b to the sheath 2b while having the sheath 2b exposed by a desired amount. That is, an end face of the pressing portion 72c in the connecting portion main frame 72 is gradually screw-connected toward a bottom face of the concave portion 74a.

Then the deforming member 73 is squished gradually by the end face of the pressing portion 72c as the connecting portion main frame 72 moves. That is, as shown in Fig. 8, the deforming member 73 contracts in a longitudinal direction and expands in a central direction. Therefore, an inner periphery of the through hole 73a in the deforming member 73 will be pressed to the outer surface of the sheath 2b to an integrated state. That is, the first attaching portion 52A is brought to as state where the first attaching portion 52A is fixed integrally to the sheath 2b.

Meanwhile, as shown in Fig. 9, the first attaching portion 52A can also be attached or removed with respect to the connecting member 71 that configures the mouth ring portion 12b, as indicted by the arrows, while having the inner periphery of the deforming member 73 being pressed to the outer surface of the sheath 2b to an integrated state, that is, while having the first attaching portion 52A in the state of being fixed integrally with respect to the sheath 2b. Thereby, the operator, etc. can insert and withdraw the sheath 2b by manual operation without having to touch unclean areas of the sheath 2b having been used in the treatment. Moreover, by attaching the first attaching portion 52A to the mouth ring portion 12b while having the first attaching portion 52A fixed integrally to a predetermined place of the sheath 2b, the tissue collecting portion 2a can be arranged at a desired place inside the treatment instrument channel 11e.

Still another configuration example of the first attaching portion will now be described with reference to Fig. 10.

As shown in Fig. 10, a connecting tube 50B has a first attaching portion 52B in an integrated part thereof. The first attaching portion 52B is configured as being detachable with respect to the connecting member 71 which is arranged integrally to the mouth ring portion 12b. The first attaching portion 52B is configured as including a connecting portion main frame 76 and a deforming holding portion 77.

The deforming holding portion 77 is a tubular elastic member including a through hole 77a, and the deforming holding portion 77 is also serving as the fixing portion main frame. A diameter of the through hole 77a is being previously set to be larger than a diameter of the sheath 2b. The deforming holding portion 77 has a protruding portion 77b at a proximal end side thereof. The protruding portion 77b is fixed to one end portion of the tube main frame 51 integrally by adhesion, for example.

The connecting portion main frame 76 is a tubular rigid member provided with a through hole 76a whose diameter is previously set to be larger than the diameter than the sheath 2b. The connecting portion main frame 76 includes a press fitting portion 76c having a taper face 76b and a grasping portion 76d of an approximately columnar shape. The grasping portion 76d has a convex portion 76e on a proximal end side. The convex portion 76e is fixed to one end portion of the deforming holding portion 77 integrally by adhesion, for example.

The taper face 76b of the press fitting portion 76c is pressed into the taper hole 71c of the press fit fixing portion 71b. Thereby, the first attaching portion 52B is arranged fixed to the mouth ring portion 12b integrally.

According to the configuration of the first attaching portion 52B, the operation at the time of attaching or removing the first attaching portion 52B with respect to the connecting member 71 of the mouth ring portion 12b will be different between a case of grasping the grasping portion 76d of the connecting portion main frame 76 and a case of grasping the deforming holding portion 77.

That is, the operator may grasp the grasping portion 76d of the connecting portion main frame 76 and press the taper face 76b into the taper hole 71c of the connecting member 71. Thereby, attachment of the first attaching portion 52B arranged at the distal end portion of the tube main frame 51 to the mouth ring portion 12b will be completed.

Meanwhile, in removing the first attaching portion 52B from the mouth ring portion 12b, the operator grasps the grasping portion 76d and removes the taper face 76b of the connecting portion main frame 76 from the taper hole 71 c against the elastic force of the connecting member 71.

Thereby, in the same way as in the case of the above-described first attaching portion 52 or the case of the above-described first attaching portion 52A shown in Fig. 7, attachment or removal of the first attaching portion 52B with respect to the mouth ring portion 12b can be done easily in one sequence of actions. Then a part of the sheath 2b can be brought to an exposed state by removing the first attaching portion 52B from the mouth ring portion 12b.

On the other hand, the operator may grasp the deforming holding portion 77 and press the taper face 76b into the taper hole 71c of the connecting member 71, or remove the taper face 76b from the taper hole 71c. At this time, the deforming holding portion 77 will be deformed as shown by chain double dashed lines due to the grasping force applied as the operator, etc. grasps the deforming holding portion 77. Then the operator, etc. will be able to hold the sheath 2b through the deforming holding portion 77. In other words, the first attaching portion 52B becomes integral with the sheath 2b.

Thereby, as shown in the above-mentioned Fig. 9, the first attaching portion 52B can be attached or removed with respect to the connecting member 71 while the first attaching portion 52B is in the state of being fixed to the sheath 2b integrally. That is, the operator can insert and withdraw the sheath 2b by manual operation without having to touch unclean areas of the sheath 2b having been used in the treatment.

The configuration of the first attaching portion 52B has a simpler structure than the configuration of the first attaching portion 52A. Accordingly, the first attaching portion can be realized with the configuration which is inexpensive.

In the above-described embodiment, the connecting tube 50 is configured as including the stretchable tube main frame 51. However, the configuration of the connecting tube is not limited to being the configuration that includes the stretchable tube main frame. That is, the tube main frame can be made rigid by using a resin member instead of the stretchable member, for instance, so as to configure a rigid connecting tube.

In the rigid connecting tube, the rigid tube main frame is provided with the first attaching portion and the second attaching portion at respective end portions thereof. In a case of letting a part of the sheath be exposed while the rigid connecting tube is in a state of being connected to the mouth ring portion of the endoscope and the connecting portion of the advancing and retracting device, the following procedures will be taken.

In a case when the first attaching portion is being attached to the mouth ring portion, first, the operator, etc. brings the first attaching portion to a detachable state with respect to the mouth ring portion. After that, the operator, etc. is to move the operation portion of the endoscope to separate the mouth ring portion from the first attaching portion. Thereby, a part of the sheath 2b will come to a sate of being exposed, and the operator, etc. will be able to grasp the sheath 2b with the fingers to advance and retract the sheath 2b by manual operation.

On the other hand, in a case when the first attaching portion is being attached to the advancing and retracting device, the first attaching portion is brought to a detachable state with respect to the connecting portion, after which the operator, etc. moves the advancing and retracting device to separate the advancing and retracting device from the first attaching portion. Thereby, a part of the sheath 2b in the proximal end side will come to a state of being exposed, by which the sheath 2b will be able to be advanced and retracted by manual operation.

In addition, in the case of using the rigid connecting tube, a configuration in which one end portion of the rigid connecting tube is connected to the mouth ring portion of the endoscope and the other end portion is arranged in the vicinity of the insertion portion inserting hole of the advancing and refracting device is also possible. Thereby, a part of the sheath can always be in a state of being exposed in between one end portion of the rigid connecting tube and the advancing and retracing device.

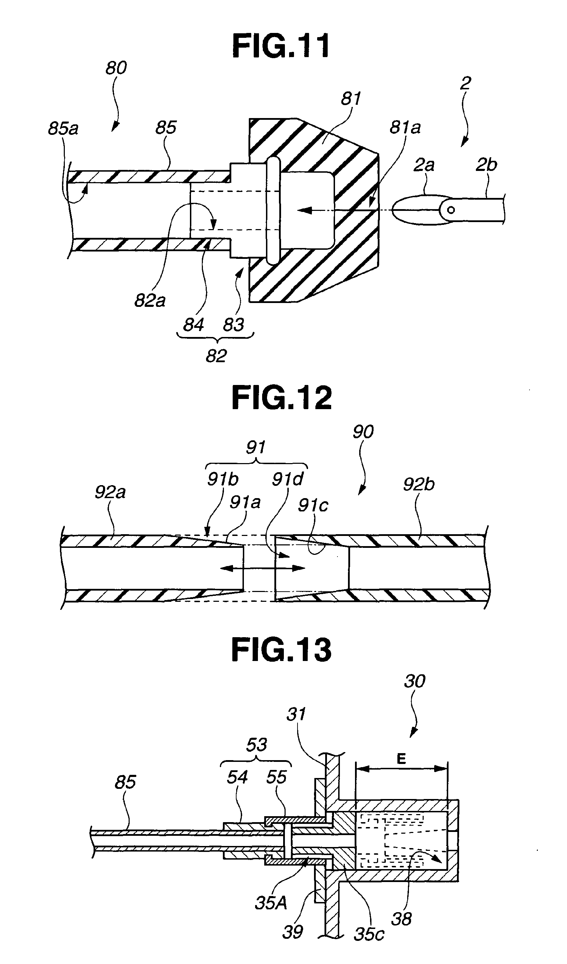

In this configuration, as shown in Fig. 11, a mouth ring configuration member 82 to which a forceps plug 81 is detachable is arranged instead of having the attaching portion arranged at the proximal end portion of the rigid connecting tube 80.

The mouth ring configuration member 82 has a through hole 82a, and is configured as including an attaching portion 83, which is configured as having approximately the same shape as the above-mentioned convex portion 12d and the mouth ring portion 12b, and a tube fixing portion 84. The tube fixing portion 84 is fixed to an end portion of a rigid tube main frame 85, which configures the rigid connecting tube 80, integrally by adhesion, for instance.

By having the mouth ring configuration member 82 arranged in the rigid connecting tube 80, the forceps plug 81 attachable to the mouth ring portion 12b of the endoscope 10 can be arranged at the mouth ring configuration member 82. Then the tissue collecting portion 2a and the sheath 2b can be introduced into an inner hole 85a of the rigid tube main frame 85 through a slit 8 1 a of the forceps plug 81 and the through hole 82a.

Thereby, the functional portion and the treatment instrument insertion portion being advanced from the advancing and retracting device can be introduced into the inner hole of the rigid connecting tube in a desired state. Accordingly, impairment in the advancing and retracting activity of the sheath conducted by the advancing and retracting device and the sheath portion touching unclean areas can be prevented without failure.

In the above-described embodiment, the tube main frame of the connecting tube or the rigid tube main frame of the rigid connecting tube is configured with a single member. However, the tube main frame can be structured as being configured with a plurality of tube frames which are separable.

A rigid tube main frame 90 configuring the rigid connecting tube, as shown in Fig. 12, has a connecting portion 91, and is configured as including a plurality of rigid tube frames 92a, 92b, ... being connected.

The connecting portion 91 is configured as including a male portion 91 b which has a taper face 91 a arranged on an outer periphery of a proximal end side of the tube frame 92a on one side and a female portion 91 d which has a taper face 91 c arranged on an inner periphery of a distal end side of the tube frame 92b on the other side. The connecting portion 91 between the tube frame 92a and the tube frame 92b is fixed integrally through press fitting.

Thereby, in the rigid connecting tube provided with the rigid tube main frame configured as connecting a plurality of rigid tube frames, by releasing a connecting state of the connecting portion in the rigid tube main frame, a part of the sheath can be exposed from the released portion.

Meanwhile, a configuration of a tube main frame for a connecting tube having elasticity in which a plurality of stretchable elastic tube frames are connected through arrangement of connecting portions is also possible.

Moreover, as shown in Fig. 13, for instance, a configuration is also possible in which a space portion 38 is provided inside the box chassis 31 of the advancing and retracting device 30, and a connecting portion 35A, where the second attaching portion 53 is attached to the space portion 38 in a slidable way, is arranged. Reference numeral 39 denotes a stopper. The stopper 39 prevents the connecting portion 35A from dropping off from the space portion 38 as a convex portion 35c of the connecting portion 35A touches the stopper 39.

Thereby, in the state where the mouth ring portion 12b and the advancing and retracting device 30 are being connected through the rigid connecting tube 80 or 90, the first attaching portion 52 may be removed from the mouth ring portion 12b or the connecting state of the connecting portion 91 may be released. At this time, the connecting portion 35A is moved inside the space portion 38 from a position indicated by a solid line to a position indicated by a dashed line by as much as the length "E", for instance.

Then a part of the sheath 2b will come to a sate of being exposed without having the endoscope 10 and the advancing and retracting device 30 being moved, whereby the operator can advance and retract the sheath 2b by manual operation.

The present invention is not limited to the above-described embodiment while various changes may be made without departing from the scope of the invention.

## Claims

1. An endoscope treatment system, comprising:
an advancing and retracing device which electromotively advances and retracts a treatment instrument insertion portion of a treatment instrument to be introduced in a treatment instrument insertion channel through a treatment instrument opening opened at a mouth ring portion of an endoscope; and
a connecting tube including a tube main frame to which the treatment instrument insertion portion is inserted and an attaching portion provided at one end portion of the tube main frame, while the attaching portion is arranged at the mouth ring portion or the treatment instrument insertion portion integrally.

2. The endoscope treatment system according to claim 1, wherein
the attaching portion includes:
an attaching portion main frame which is a tubular member arranged fixed to the tube main frame integrally;
a pair of holding members each of which being provided with a first holding portion which has a nail-like shape and is arranged locked at the mouth ring portion and a second holding portion which has a peripheral surface portion to be arranged coherent to an outer surface of the treatment instrument insertion portion; and
an urging member which urges the holding portions of the holding members in a central axis direction.

3. The endoscope treatment system according to claim 1, wherein
the attaching portion is detachable with respect to a connecting member arranged at the mouth ring portion integrally, the attaching portion including:
a fixing portion main frame which is a tubular rigid member provided with a concave portion and which is being arranged fixed to the tube main frame integrally;
a deforming member which is configured with a tubular elastic member and which is arranged inside the concave portion; and
a connecting portion main frame which is configured with a tubular rigid member, and which includes a pressing portion which is capable of advancing and retracting inside the concave portion and deforming the deforming member by pressing and a press fitting portion which is pressed into the connecting member.

4. The endoscope treatment system according to claim 1, wherein
the attaching portion is detachable with respect to a connecting member arranged at the mouth ring portion integrally, the attaching portion including:
a deforming holding portion which is a tubular elastic member arranged fixed to the tube main frame integrally; and
a connecting portion main frame arranged fixed to the deforming holding portion integrally, the connecting portion main frame being provided with a press fitting portion which is pressed into the connecting member and being configured with a tubular rigid member.

5. The endoscope treatment system according to claim 1, wherein
the tube main frame is a stretchable elastic member.

6. The endoscope treatment system according to claim 1, wherein
the tube main frame is a rigid member.

7. The endoscope treatment system according to claim 5 or 6, wherein
the tube main frame has a connecting portion.
